(19) Europäisches Patentamt
European Patent Office
Office européen des brevets

(11) Veröffentlichungsnummer: **0 034 253**
**B1**

# (12) EUROPÄISCHE PATENTSCHRIFT

(45) Veröffentlichungstag der Patentschrift: **18.07.84**

(51) Int. Cl.³: **C 07 C 121/75**, C 07 C 101/72, C 07 C 99/10, C 07 B 19/00

(21) Anmeldenummer: **81100259.1**

(22) Anmeldetag: **15.01.81**

## (54) 4-tert.-Butoxyphenylglycinnitril sowie Verfahren zur Herstellung von D-(-)- und L-(+)-4-Hydroxyphenylglycin.

(30) Priorität: **25.01.80 DE 3002543**

(43) Veröffentlichungstag der Anmeldung: **26.08.81 Patentblatt 81/34**

(45) Bekanntmachung des Hinweises auf die Patenterteilung: **18.07.84 Patentblatt 84/29**

(84) Benannte Vertragsstaaten: **BE CH DE FR GB IT LI NL**

(56) Entgegenhaltungen: **EP - A - 0 001 060**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Degner, Dieter, Dr., Kurpfalzstrasse 8, D-6701 Dannstadt-Schauernheim (DE)**
Erfinder: **Pander, Hans Joachim, Hochdorfer Strasse 19, D-6701 Roedersheim-Gronau (DE)**
Erfinder: **Siegel, Hardo, Dr., Hans-Purrmann-Allee 25, D-6720 Speyer (DE)**

**EP 0 034 253 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft die neue Verbindung 4-tert.-Butoxyphenylglycinnitril (I)

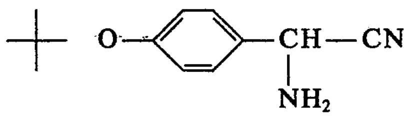

(I)

sowie hiervon ausgehend ein verbessertes Verfahren zur Herstellung von D-(−)-4-Hydroxyphenylglycin (IIa) und L-(+)-4-Hydroxyphenylglycin (IIb).

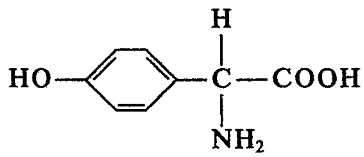

(IIa)

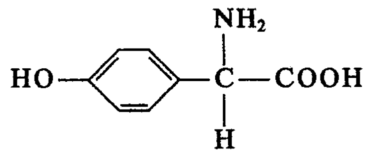

(IIb)

Aus der EP-OS 0 001 060 ist es bekannt, die optischen Isomeren des Phenylglycins (IIc)

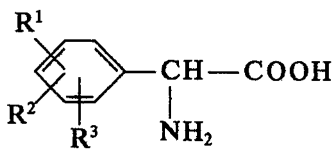

(IIc)

wobei die Reste $R^1$ bis $R^3$ Wasserstoff oder u. a. Hydroxyl, Alkyl- oder Alkoxygruppen sein können, auf die Weise herzustellen, indem man die entsprechenden DL-Phenylglycinnitrile Ic

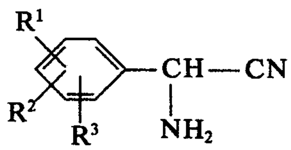

(Ic)

in vorzugsweise methanolischer Lösung sowie in Gegenwart einer Carbonylverbindung mit L-(+)-Weinsäure umsetzt, das hierbei auskristallisierende schwerer lösliche diastereomere L-(+)-Tartrat abtrennt und danach zur Säure IIc hydrolysiert. Während der Bildung des Tartrates findet hierbei infolge der Anwesenheit der Carbonylverbindung eine Racemisierung desjenigen optischen Isomeren von Ic statt, welches das leichter lösliche diastereomere Tartrat bildet, wogegen das Isomere, welches das schwerer lösliche Tartrat bildet, der Racemisierung durch Kristallisation des Tartrates entzogen wird. Dies bedeutet, daß zumindest theoretisch das gesamte DL-Ic in das schwerer lösliche L-(+)-Tartrat (entweder des D-Ic oder des L-Ic) überführt wird. Aus dem 4-Hydroxyderivat von Ic erhält man hierbei die Verbindung IIa, die eine große Bedeutung für die Synthese halbsynthetischer Antibiotica hat.

Die Gesamtsynthese von IIa, die von 4-Hydroxybenzaldehyd ausgeht und in ihren Teilschritten aus dem in der EP-OS cit abgehandelten Stand der Technik bekannt ist,

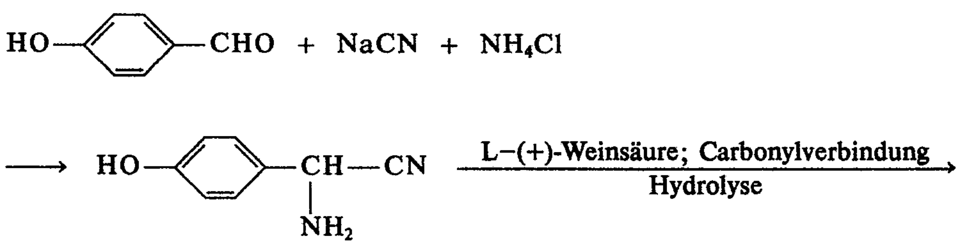


(IIa)

ist jedoch wegen unzureichenden Ausbeuten wirtschaftlich unbefriedigend.

Aus diesem Grunde wurde es bisher bevorzugt, vom Methyläther des 4-Hydroxybenzaldehyds auszugehen und den Äther im letzten Syntheseschritt wieder zur freien Hydroxyverbindung IIa zu spalten. Da die hierzu erforderlichen Reaktionsbedingungen jedoch relativ scharf sind, läßt sich eine teilweise Racemisierung von IIa praktisch nicht vermeiden.

Der Erfindung lag daher die Aufgabe zugrunde, die Verbindung IIa unter Vermeidung dieses Nach-

teils technisch und wirtschaftlich besser zugänglich zumachen. Diese Aufgabe erstreckt sich auch auf die Herstellung der antipodischen Verbindung IIb, die ebenfalls ein wichtiges Zwischenprodukt für Arzneimittel ist.

Demgemäß wurde gefunden, daß man das D-(—)-4-Hydroxyphenylglycin (IIa) und das L-(+)-4-Hydroxyphenylglycin (IIb)

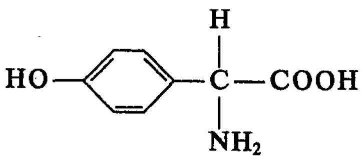

(IIa)

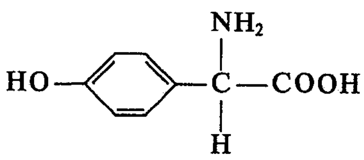

(IIb)

auf vorteilhafte Weise erhält, wenn man DL-4-tert.-Butoxyphenylglycinnitril (I)

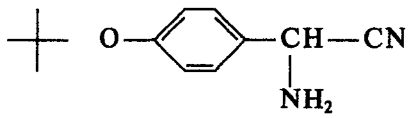

(I)

in an sich bekannter Weise in alkoholischer Lösung sowie in Gegenwart einer Carbonylverbindung mit etwa der äquimolaren Menge L-(+)-Weinsäure bzw. D-(—)-Weinsäure umsetzt und das hierbei in kristalliner Form anfallende D-(—)-I-L-(+)-Tartrat bzw. L-(+)-I-D-(—)-Tartrat bei 20 bis 110° C in saurer wäßriger Lösung unter Abspaltung der L-(+)- bzw. D-(—)-Weinsäure, Hydrolyse der Nitrilgruppe und hydrolytischer Abspaltung der tert.-Butylgruppe in IIa bzw. IIb überführt.

Es wurde weiterhin gefunden, daß sich bei dieser Reaktion das Methylglyoxaldimethylacetal (III)

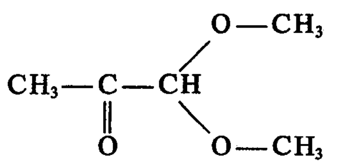

(III)

besonders gut als Carbonylverbindung eignet.

Die für das erfindungsgemäße Verfahren benötigte Ausgangsverbindung DL-I ist neu. Sie ist in an sich bekannter Weise durch Umsetzung von 4-tert.-Butoxybenzaldehyd mit wasserfreier Blausäure und danach mit wasserfreiem Ammoniak bei (—20) bis 30° C erhältlich.

Die anschließende, unter Stereoisomerisierung verlaufende Umsetzung mit L-(+)-Weinsäure bzw. D-(—)-Weinsäure wird in alkoholischer, insbesondere methanolischer Lösung vorgenommen, und zwar entweder in Methanol allein oder vorzugsweise in einem Lösungsmittelgemisch, welches Methanol enthält. Gut bewährt haben sich beispielsweise Gemische mit Ethylacetat, Benzol, Toluol, Methylenchlorid und Dichlorethan mit jeweils 10 bis 80 Vol.-% Methanolgehalt.

Die Menge dieser Lösungsmittel beträgt etwa 2 bis 8 l pro kg I.

Als Carbonylverbindungen eignen sich prinzipiell alle Aldehyde und Ketone und auch deren niedere Dialkylacetale, sofern die Carbonylgruppe bzw. die Acetalgruppierung nicht sterisch behindert wird. Beispiele für derartige Carbonylverbindungen, deren Verwendung für die Stereoisomerisierung von Phenylglycin, Phenylglycinestern, Phenylglycinnitril und deren kernsubstituierten Derivaten bekannt ist, sind Aceton, Benzaldehyd, Cyclohexanon, Methylethylketon, n-Butyraldehyd und Methylisobutylketon.

Besonders gute Ergebnisse bezüglich einer weitgehenden Steroisomerisierung erzielt man im vorliegenden Fall mit der Verbindung III, welche für die Zwecke der oben genannten Synthesen bisher noch nicht vorgeschlagen wurde. Die gute Eignung von III ist vor allem deshalb bemerkenswert, als es sich um eine bifunktionelle Carbonylverbindung handelt. Da anzunehmen ist, daß die Phenylglycinderivate mit den Carbonylverbindungen intermediär Schiffsche Basen bilden, die besonders leicht racemisieren, war im Falle von bifunktionellen Carbonylverbindungen eine beidseitige Umsetzung mit den Phenylglycinverbindungen und damit eine Störung des Racemisierungsmechanismus zu befürchten.

Man verwendet die Carbonylverbindungen allgemein vorzugsweise in Mengen von 1 bis 4 mol pro mol I, wobei die Verbindungen III den Vorteil bieten, daß man hier mit geringeren Mengen — etwa 0,1 bis 0,5 mol — auskommt.

Die unter gleichzeitiger Bildung des festen D-(—)-I-L-(+)-Tartrates verlaufende Reaktion, für die man die L-(+)-Weinsäure in etwa äquimolaren Mengen zum DL-I einsetzt, beansprucht allgemein, d. h. unter Mitverwendung beliebiger Carbonylverbindungen, etwa 2 bis 6 Stunden. Bei Verwendung von III als Carbonylverbindung läßt sich die Reaktionszeit auf etwa eine Stunde senken. Das gleiche gilt naturgemäß für die spiegelbildliche Verbindung, das L-(+)-I-D-(—)-Tartrat.

Das auskristallisierte Tartrat wird sodann abgetrennt und in verdünnter wäßriger Säure, z. B. verdünnter Salzsäure oder Schwefelsäure, gelöst und auf 20 bis 110°C erwärmt. Hierbei wird die Weinsäure in Freiheit gesetzt, die Nitrilgruppe in die Carboxylgruppe überführt und die tert.-Butylgruppe unter Bildung von tert.-Butanol oder z. B. tert.-Butylchlorid und der freien phenolischen Hydroxylgruppe von IIa bzw. IIb hydrolytisch abgespalten.

Die Aufarbeitung des Hydrolysegemisches auf die gewünschten Verbindungen IIa bzw. IIb erfolgt dann wie üblich. Man erhält IIa bzw. IIb, bezogen auf I, in Ausbeuten von etwa 60 bis 75%.

## Beispiel 1

### Herstellung von (DL)-p-tert.-Butoxyphenylglycinnitril (I)

#### a) Herstellung von p-tert.-Butoxybenzaldehyd-cyanhydrin

Eine Lösung aus 190 g (7 mol) wasserfreier Blausäure, 2 g N,N-Dimethylethanolamin als basischem Kondensationsmittel und 400 g Diethylether wurde unter Rühren bei 0 bis 5°C portionsweise mit 1212 g (6,8 mol) p-tert.-Butoxybenzaldehyd versetzt und danach noch eine Stunde bei (−10)°C gehalten. Das als Kristallbrei ausgefallene Cyanhydrin wurde abgetrennt und getrocknet; Smp. 73 bis 74°C; Ausbeute 92%.

#### b) Herstellung von I

Eine Mischung aus 51,3 g (0,25 mol) des Cyanhydrins und 68 g (4 mol) wasserfreiem Ammoniak wurde in einem Autoklaven 24 h bei 20 bis 30°C gerührt. Hierbei bildete sich I als kristallines Reaktionsprodukt in einer Ausbeute von 94%, Smp. 50 bis 51°C. Das Rohprodukt, dessen Reinheit demgemäß 94% betrug, wurde ohne weitere Reinigung für die weiteren Umsetzungen verwendet.

## Beispiel 2

### Herstellung von D-(−)-I-L-(+)-Tartrat mit Aceton als Carbonylverbindung

86,8 g (0,42 mol) (DL)-4-tert.-Butoxyphenylglycinnitril (I) und 64 g (0,42 mol) L-(+)-Weinsäure wurden zusammen mit 24 g (0,42 mol) Aceton 5 h mit einer Mischung aus 175 ml Methanol und 520 ml Ethylacetat bei 40°C gerührt. Nach Abkühlung auf 20°C bildete sich ein Kristallbrei, der abgetrennt und getrocknet wurde. Die Ausbeute an dem Tartrat betrug 78%.

$[\alpha]_D^{20} = +32,0°$ (c=1; $H_2O$); dies entspricht einer optischen Ausbeute der D-(−)-I-Komponente des Tartrates von 94%.

## Beispiel 3

### Herstellung von D-(−)-I-L-(+)-Tartrat mit III als Carbonylverbindung

Eine Lösung von 102 g (0,5 mol) I in 490 ml Methylenchlorid wurde mit einer Lösung aus 75 g (0,5 mol) 1-(+)-Weinsäure, 14,5 g (0,13 mol) Methylglyoxaldimethylacetal und 165 ml Methanol versetzt, zwei Stunden unter Rückflußkühlung erhitzt und danach auf 20°C abgekühlt. Die Ausbeute an dem hierbei ausgefallenen Salz betrug 86%.

$[\alpha]_D^{20} = +32,3°$ (c=1; $H_2O$); dies entspricht einer optischen Reinheit der D-(−)-I-Komponente des Tartrates von 98%.

## Beispiel 4

### Herstellung von L-(+)-I-D-(−)-Tartrat

Diese Verbindung wurde analog Beispiel 3 aus I und D-(−)-Weinsäure hergestellt; Ausbeute 85%.

$[\alpha]_D^{20} = -31,3°$ (c=1; $H_2O$); dies entspricht einer optischen Reinheit der L-(+)-I-Komponente des Tartrates von 95%.

Beispiel 5

Herstellung von D-(−)-4-Hydroxyphenylglycin (IIa)

33 g (rd. 0,2 mol) des nach Beispiel 2 hergestellten Tartrates wurden mit 150 ml 20gew.-%iger Salzsäure erhitzt, wobei tert.-Butylchlorid abdestillierte. Nach einstündigem weiteren Erhitzen unter Rückflußkühlung wurde das Reaktionsgemisch abgekühlt, mit Aktivkohle versetzt und filtriert.

Danach wurde die Lösung mit 50gew.-%iger Natronlauge auf pH 6,5 bis 7,0 gestellt, wonach das Verfahrensprodukt auskristallisierte. Dieses wurde sodann abgetrennt und getrocknet. Die Ausbeute an IIa betrug 79%, bezogen auf das eingesetzte Tartrat.

$[\alpha]_D^{20} = -151°$ (1 n HCl); dies entspricht einer optischen Reinheit an IIa von 94%.

Das nach Beispiel 3 hergestellte Tartrat lieferte in gleicher Weise die Verbindung IIa in 82%iger Ausbeute und in 98%iger optischer Reinheit; $[\alpha]_D^{20} = -156°$ (1 n HCl).

Beispiel 6

Herstellung von L-(+)-4-Hydroxyphenylglycin (IIb)

Diese Verbindung wurde analog Beispiel 5 aus dem Tartrat gemäß Beispiel 4 hergestellt. Die Ausbeute betrug 76%, die optische Reinheit 95%.

$[\alpha]_D^{20} = +152°$ (1 n HCl).

**Patentansprüche**

1. 4-tert.-Butoxyphenylglycinnitril (I).

2. Verfahren zur Herstellung von D-(−)-4-Hydroxyphenylglycin (IIa) und L-(+)-4-Hydroxyphenylglycin (IIb)

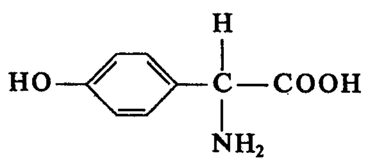

(IIa)

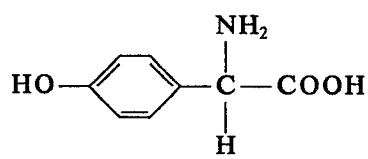

(IIb)

dadurch gekennzeichnet, daß man DL-4-tert.-Butoxyphenylglycinnitril (I)

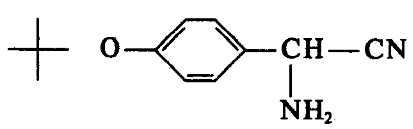

(I)

in an sich bekannter Weise in alkoholischer Lösung sowie in Gegenwart einer Carbonylverbindung mit etwa der äquimolaren Menge L-(+)-Weinsäure bzw. D-(−)-Weinsäure umsetzt und das hierbei in kristalliner Form anfallende D-(−)-I-L-(+)-Tartrat bzw. L-(+)-I-D-(−)-Tartrat bei 20 bis 110°C in saurer wäßriger Lösung unter Freisetzung der L-(+)-Weinsäure bzw. der D-(−)-Weinsäure, Hydrolyse der Nitrilgruppe und hydrolytischer Abspaltung der tert.-Butylgruppe in IIa bzw. IIb überführt.

3. Verfahren nach Anspruch 2, dadurch gekennzeichnet, daß man als Carbonylverbindung Methylglyoxaldimethylacetat (III)

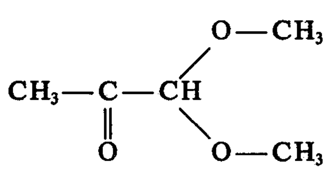

(III)

verwendet.

**Claims**

1. 4-tert.-Butoxyphenylglycinonitrile (I).

2. A process for the preparation of D-(−)-4-hydroxyphenylglycine (IIa) and L-(+)-4-hydroxyphenylglycine (IIb)

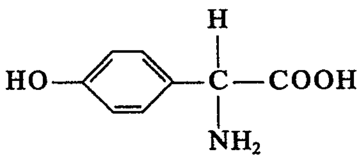

(IIa)

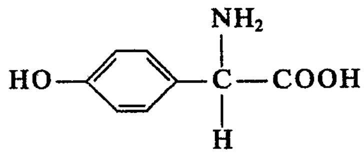

(IIb)

wherein DL-4-tert.-butoxyphenylglycinonitrile (I)

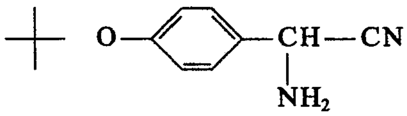

(I)

is reacted, in a conventional manner, in alcoholic solution and in the presence of a carbonyl compound, with about the equimolar amount of L-(+)-tartaric acid or D-(−)-tartaric acid and the D-(−)-I-L-(+)-tartrate or L-(+)-I-D-(−)-tartrate thereby obtained in crystalline form is converted into IIa or IIb in aqueous acis solution at 20−110°C by liberating the L-(+)-tartaric acid or D-(−)-tartaric acid, hydrolyzing the nitrile group and hydrolytically splitting off the tert.-butyl group.

3. A process as claimed in claim 2, wherein methylglyoxal dimethylacetal (III)

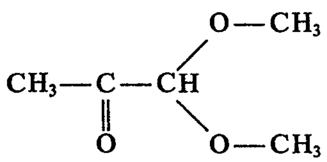

(III)

is used as the carbonyl compound.

**Revendications**

1. 4-tertiobutoxyphénylglycine-nitrile (I).

2. Procédé pour la préparation de D-(−)-4-hydroxyphénylglycine (IIa) et de L-(+)-4-hydroxyphénylglycine (IIb)

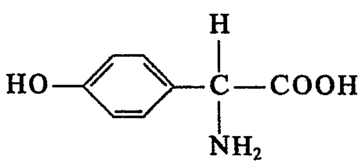

(IIa)

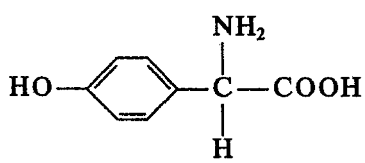

(IIb)

caractérisé en ce qu'on fait réagir le DL-4-tertiobutoxyphénylglycine-nitrile (I)

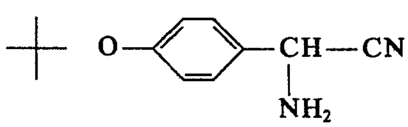

(I)

de façon connue en soi, en solution alcoolique ainsi qu'en présence d'un composé carbonylé, avec à peu près la quantité équimolaire d'acide tartrique L-(+) ou d'acide tartrique D-(−) et on transforme en IIa ou IIb le L-(+)-tartrate de D-(−)-I ou le D-(−)-tartrate de L-(+)-I, obtenu dans ces conditions à l'état cristallin, à une température de 20 à 110°C en solution aqueuse acide, avec libération de l'acide tartrique L-(+) ou de l'acide tartrique D-(−), hydrolyse du groupe nitrile et dissociation hydrolytique du groupe butyle tertiaire.

3. Procédé selon la revendication 2, caractérisé en ce qu'on utilise, en tant que composé carbonylé,

un diméthylacétal de méthylglyoxal (III)

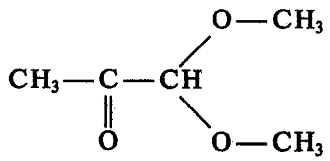

(III)